# EUROPEAN PATENT APPLICATION

(11) **EP 4 273 872 A1**
(43) Date of publication of application: **08.11.2023**
(21) Application number: 22209026.8
(22) Date of filing: 23.11.2022
(51) Int. Cl.: G16H 20/10, G16H 20/30, G16H 50/30, G16H 80/00, A61B 5/00, A61B 5/024

(54) **APPARATUS FOR DIFFERENTIALLY CALCULATING TARGET HEART RATES OF DIFFERENT USERS**

(30) Priority: 07.05.2022 CN 202210489237
(71) Applicant: Chengdu Shangyi Information Technology Co., Ltd., Chengdu, Sichuan 610000 (CN)
(72) Inventor: LEI, Chi, Chengdu, 610000 (CN); CHEN, Xi, Chengdu, 610000 (CN); ZHAO, Guo, Chengdu, 610000 (CN); ZENG, Ling, Chengdu, 610000 (CN); FAN, Yi, Chengdu, 610000 (CN)
(74) Representative: M. Zardi & Co S.A.

(57) **Abstract**

The present disclosure relates to an apparatus for differentially calculating target heart rates of different users, including: a user client, including a health data acquisition module, a medication data acquisition module, and a user-side target heart rate display module; a heart rate detection device, worn on a user and including an exercise test data acquisition module; a server, including a data input module and a core processor; and a doctor client, including a data sending module and a doctor-side target heart rate display module.

## Description

### TECHNICAL FIELD

The present disclosure relates to the field of healthcare informatics, and in particular, to an apparatus for differentially calculating target heart rates of different users.

### BACKGROUND ART

As living standards improve, exercise and fitness gradually become important demands in people's life. Lacking of physical activities (PAs) is closely related to occurrences, development, and progression of various diseases. As early as 1992, the American Heart Association (AHA) published a report to list lacking of PAs as the fourth most modifiable risk factor for heart disease. A report published by the World Health Organization (WHO) in 2002 listed lacking of PAs as one of the ten major causes of death in developed countries. Numerous studies have found that health problems caused by lacking of PAs, which directly related to decreased cardiopulmonary endurance. Improving cardiopulmonary endurance can reduce occurrences of related diseases, slow down disease development, prevent and treat complications. Cardiopulmonary endurance is an ability of a human body to take in and utilize oxygen, reflecting an overall health condition of the human body in respiratory, circulatory, musculoskeletal, and metabolic systems. In 2016, the AHA proposed that cardiopulmonary endurance should be regarded as the fifth vital sign in addition to respiration, body temperature, pulse, and blood pressure and become a routine metric concerned by medical service providers. In 2018, the AHA re-emphasized serious disease burden due to lacking of PAs and the needs of performing routine chronic disease assessment and improving cardiopulmonary endurance.

The main way to improve cardiopulmonary endurance is scientific exercise. In 2007, the American College of Sports Medicine (ACSM) and the American Medical Association (AMA) jointly launched the "Exercise is medicine" project based on the core idea of promoting health through scientific exercise, proposing that correctly formulating and using exercise prescriptions enables human bodies to benefit most from exercise and is the key to prevention and treatment of chronic diseases. In fact, as early as 1969, the WHO formally adopted the concept of "exercise prescription". An exercise frequency, intensity, time, type, volume, and progression suitable for an athlete are determined based on a fitness level and health condition of the athlete. As a clinician prescribes different medications based on a patient's condition, ACSM later referred to the frequency, intensity, time, type, volume, and progression as the FITT-VP principle of the exercise prescription. Based on evidence-based medicine, ACSM's Guidelines for Exercise Testing and Prescription is developed with basic principles of exercise testing as basic content, and updated to the tenth edition in 2017, which is the ground truth of current global guidelines for exercise rehabilitation. Exercise prescription is different from physical activity. Exercise prescription is designed according to age, gender, living habits, health conditions, and physical fitness levels, to ensure safety and effectiveness of exercise.

As science and technology rapidly developing, the wearable devices and mobile networks provides a reliable basis for scientific and appropriate exercise management. In the field of exercise physiology, appropriate aerobic exercise is considered to be an important metric to obtain an ideal effect of exercise fitness. If a person perform exercise at the heart rate range for a specific period of time, the exercise will be good at improving cardiopulmonary endurance. If the heart rate is too low, the exercise effect is poor. If the heart rate is too high, the person may put himself at risk.

Therefore, how to calculate target heart rate range based on user's conditions to formulate appropriate exercise prescriptions so as to achieve effective exercise is close to actual requirements, and is a technical problem.

### SUMMARY

An objective of the present disclosure is to provide an apparatus for calculating target heart rates of users according to the user's own health conditions.

In order to achieve the above objective, the present disclosure provides the following technical solutions.

An apparatus for calculating target heart rates of different users is provided. The apparatus includes a user client, a heart rate detection device, a server, and a doctor client. The user client includes a health data acquisition module, a medication data acquisition module, a user-side target heart rate display module, and an exercise test data acquisition module. The server includes a data input module and a core processor. The doctor client includes a data sending module and a doctor-side target heart rate display module.

The health data acquisition module is configured to acquire health information of the user. The health information includes age and health conditions of the user. The health condition includes a disease diagnosis of the user.

The medication data acquisition module is configured to acquire medication information of the user. The medication information includes whether the user uses a medication which can slow down the heart rate.

The exercise test data acquisition module is configured to acquire heart rate information of the user. The heart rate information includes whether the user has had a resting heart rate test and results of the test.

The data input module is configured to input the health information, the medication information, and the heart rate information of the user to the core processor.

The core processor is configured to calculate a target heart rate range of the user based on the health information, the medication information, and the heart rate information of the user and send the target heart rate range to the doctor client.

The doctor-side target heart rate display module is configured to display the target heart rate range on the doctor client.

The data sending module is configured to send the target heart rate range from the doctor client to the user client.

The user-side target heart rate display module is configured to display the target heart rate range on the user client.

Optionally, the core processor may specifically include:
a maximum heart rate calculation submodule, configured to calculate a maximum heart rate of the user based on the age of the user;
an expected intensity determining submodule, configured to determine an expected intensity of recommended exercise of the user based on the health condition of the user; and
a target heart rate range calculation submodule, configured to calculate the target heart rate range of the user based on the maximum heart rate, the expected intensity of the recommended exercise, the medication information, and the heart rate information of the user and send the target heart rate range to the doctor client.

Optionally, the maximum heart rate calculation submodule may specifically include:
a first maximum heart rate calculation unit, configured to: if the age of the user is 4 to 34, calculate the maximum heart rate *HRₘₐₓ* of the user by using the following formula: *HRₘₐₓ* = 216.6 - 0.84 × *Age;* and
a second maximum heart rate calculation unit, configured to: if the age of the user is greater than 34, calculate the maximum heart rate *HRₘₐₓ* of the user by using the following formula: *HRₘₐₓ* = 220 - *Age.*

Optionally, the expected intensity determining submodule may specifically include:
a first expected intensity determining unit, configured to: if the health condition of the user is poor, determine the expected intensity *ES* of the recommended exercise of the user as a low intensity to a medium intensity; where the low intensity is greater than or equal to 30% and less than 40%; and the medium intensity is greater than or equal to 40% and less than 60%; and
a second expected intensity determining unit, configured to: if the health condition of the user is normal, determine the expected intensity *ES* of the recommended exercise of the user as the medium intensity to a high intensity; where the high intensity is greater than or equal to 60% and less than 90%.

Optionally, the target heart rate range calculation submodule may specifically include:
a first target heart rate range calculation unit, configured to: if the heart rate information of the user is that the resting heart rate is not tested and the medication information is that no medication for slowing down the heart rate is used, calculate a target heart rate *HR_{target}* of the user based on the maximum heart rate *HRₘₐₓ* and the expected intensity *ES* of the recommended exercise of the user by using the following formula: *HR_{target} = HRₘₐₓ* × *ES*; where a value range of the target heart rate *HR_{target}* constitutes the target heart rate range.

Optionally, the target heart rate range calculation submodule may further include:
a second target heart rate range calculation unit, configured to: if the heart rate information of the user is that the resting heart rate is not tested and the medication information is that the medication for slowing down the heart rate is used, remind, through the user-side target heart rate display module, the user to test the resting heart rate.

Optionally, the target heart rate range calculation submodule may further include:
a third target heart rate range calculation unit, configured to: if the heart rate information of the user is that the resting heart rate is tested and the medication information is that no medications for slowing down the heart rate is used, calculate the target heart rate *HR_{target}* of the user based on the maximum heart rate *HRₘₐₓ*, the expected intensity *ES* of the recommended exercise, and the resting heart rate (RHR) of the user by using the following formula: *HR_{target} =* (*HRₘₐₓ* - *RHR)* × *ES* + *RHR*; where the value range of the target heart rate *HR_{target}* constitutes the target heart rate range.

Optionally, the target heart rate range calculation submodule may further include:
a fourth target heart rate range calculation unit, configured to: if the heart rate information of the user is that the resting heart rate is tested and the medication information is that the medication for slowing down the heart rate is used, calculate the target heart rate *HR_{target}* of the user based on the maximum heart rate *HRₘₐₓ*, the expected intensity *ES* of the recommended exercise, and the resting heart rate *RHR* of the user by using the following formula: *HR_{target}* = (*HRₘₐₓ* - *RHR)* × (*ESₘᵢₙ* - 10%, *ESₘₐₓ* - 5%) + RHR; where *ESₘᵢₙ* represents a lower limit of the expected intensity *ES* of the recommended exercise, and *ESₘₐₓ* represents an upper limit of the expected intensity *ES* of the recommended exercise; and the value range of the target heart rate *HR_{target}* constitutes the target heart rate range.

Based on specific embodiments provided in the present disclosure, the present disclosure has the following technical effects:

The present disclosure provides the apparatus for differentially calculating target heart rates of different users, including: the user client, the heart rate detection device, the server, and the doctor client. The user client includes the health data acquisition module, the medication data acquisition module, and the user-side target heart rate display module. The heart rate detection device is worn on the user. The heart rate detection device includes the exercise test data acquisition module. The server includes the data input module and the core processor. The doctor client includes the data sending module and the doctor-side target heart rate display module. The application in the present disclosure serves as both an exercise rehabilitation execution platform and a data acquisition tool. The server serves as both a platform for processing data and a database for storing data. The user fills in the health information and the medication information through the user client, and wears the heart rate detection device to detect the heart rate information. The data input module inputs the information to the core processor. The core processor uses the corresponding formula to calculate the target heart rate range of the user based on the information (including the age, the health condition, whether the resting heart rate is tested in an exercise test, whether the medication for slowing down the heart rate is used, and the like) of the user. Target exercise heart rate ranges can be accurately and personalized recommended based on different user conditions.

### BRIEF DESCRIPTION OF THE DRAWINGS

To describe the technical solutions in embodiments of the present more clearly, the accompanying drawings required in the embodiments will be briefly described below. Apparently, the accompanying drawings in the following description show merely some embodiments of the present disclosure, and other drawings can be derived from these accompanying drawings by those of ordinary skill in the art without creative efforts.
FIG. 1 is a schematic structural diagram of an apparatus for differentially calculating target heart rates of different users according to an embodiment of the present disclosure; and
FIG. 2 is a schematic diagram of an application process of an apparatus for calculating target heart rates of different users according to an embodiment of the present disclosure.

### DETAILED DESCRIPTION OF THE EMBODIMENTS

The technical solutions in the embodiments of the present disclosure will be described below clearly and completely with reference to the accompanying drawings in the embodiments of the present disclosure. Apparently, the described embodiments are merely some rather than all of the embodiments of the present disclosure. All other embodiments obtained by those of ordinary skill in the art based on the embodiments of the present disclosure without creative efforts shall fall within the protection scope of the present disclosure.

An objective of the present disclosure is to provide an apparatus for differentially calculating target heart rates of different users to differentially calculate target heart rate ranges based on different user conditions.

To make the above-mentioned objective, features, and advantages of the present disclosure clearer and more comprehensible, the present disclosure will be further described in detail below in conjunction with the accompanying drawings and specific embodiments.

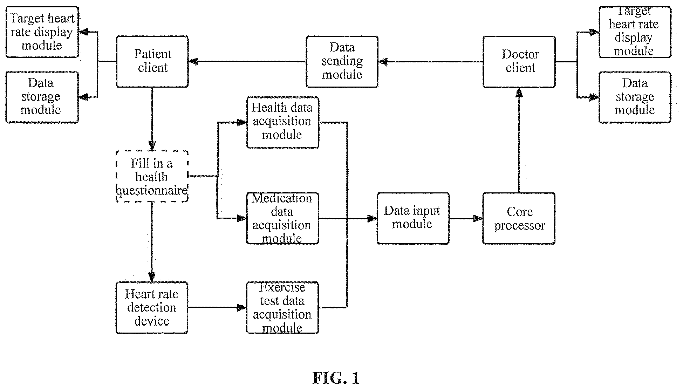

FIG. 1 is a schematic structural diagram of an apparatus for differentially calculating target heart rates of different users according to an embodiment of the present disclosure. Referring to FIG. 1, the apparatus for differentially calculating target heart rates of different users in the present disclosure includes a user client (namely, a patient application shown in FIG. 1, also referred to as a user end), a heart rate detection device, a server, and a doctor client (also referred to as a doctor end). The user client includes a health data acquisition module, a medication data acquisition module, and a user-side target heart rate display module. The heart rate detection device (including but not limited to a device with a heart rate detection function, such as a heart rate monitor, a smart watch, or a smart band) is worn on a user. The heart rate detection device includes an exercise test data acquisition module. The server includes a data input module and a core processor. The doctor client includes a data sending module and a doctor-side target heart rate display module.

The health data acquisition module is configured to acquire health information of the user. The health information includes but is not limited to age and health conditions. The health conditions include but are not limited to disease diagnoses of the user. The medication data acquisition module is configured to acquire medication information of the user. The medication information includes whether the user uses a medication for slowing down heart rate. The exercise test data acquisition module is configured to acquire heart rate information of the user. The heart rate information includes whether the user tests a resting heart rate and the resting heart rate of the user if the user tests the resting heart rate.

The data input module is configured to input the health information, the medication information, and the heart rate information of the user to the core processor. The core processor is configured to calculate a target heart rate range of the user based on the health information, the medication information, and the heart rate information of the user and send the target heart rate range to the doctor client.

The doctor-side target heart rate display module is configured to display the target heart rate range on the doctor client. The data sending module is configured to send the target heart rate range from the doctor client to the user client. The user-side target heart rate display module is configured to display the target heart rate range on the user client.

The core processor may specifically include: a maximum heart rate calculation submodule, configured to calculate a maximum heart rate of the user based on the age of the user; an expected intensity determining submodule, configured to determine an expected intensity of recommended exercise of the user based on the health condition of the user; and a target heart rate range calculation submodule, configured to calculate the target heart rate range of the user based on the maximum heart rate, the expected intensity of the recommended exercise, the medication information, and the heart rate information of the user and send the target heart rate range to the doctor client.

The maximum heart rate calculation submodule may specifically include:
a first maximum heart rate calculation unit, configured to: if the age *Age* of the user ranges from 4 to 34, calculate the maximum heart rate *HRₘₐₓ* of the user by using the following formula: *HRₘₐₓ =* 216.6 - 0.84 × *Age*; and
a second maximum heart rate calculation unit, configured to: if the *age Age* of the user is greater than 34, calculate the maximum heart rate *HRₘₐₓ* of the user by using the following formula: *HRₘₐₓ =* 220 *- Age.*

The expected intensity determining submodule may specifically include:
a first expected intensity determining unit, configured to: if the health condition of the user is poor, determine the expected intensity *ES* of the recommended exercise of the user as a low intensity to a medium intensity; where the low intensity is greater than or equal to 30% and less than 40%; and the medium intensity is greater than or equal to 40% and less than 60%; and
a second expected intensity determining unit, configured to: if the health condition of the user is normal, determine the expected intensity *ES* of the recommended exercise of the user as the medium intensity to a high intensity; where the high intensity is greater than or equal to 60% and less than 90%.

The target heart rate range calculation submodule may specifically include:
a first target heart rate range calculation unit, configured to: if the heart rate information of the user is that the resting heart rate is not tested and the medication information is that no medication for slowing down the heart rate is used, calculate a target heart rate *HR_{target}* of the user based on the maximum heart rate *HRₘₐₓ* and the expected intensity *ES* of the recommended exercise of the user by using the following formula: *HR_{target}* = *HRₘₐₓ* × *ES*; where a value range of the target heart rate *HR_{target}* constitutes the target heart rate range;
a second target heart rate range calculation unit, configured to: if the heart rate information of the user is that the resting heart rate is not tested and the medication information is that the medication for slowing down the heart rate is used, remind, through the user-side target heart rate display module, the user to test the resting heart rate;
a third target heart rate range calculation unit, configured to: if the heart rate information of the user is that the resting heart rate is tested and the medication information is that no medication for slowing down the heart rate is used, calculate the target heart rate *HR_{target}* of the user based on the maximum heart rate *HRₘₐₓ*, the expected intensity *ES* of the recommended exercise, and the resting heart rate *RHR* of the user by using the following formula: *HR_{target}* = (*HRₘₐₓ* - *RHR*) × *ES* + *RHR;* where the value range of the target heart rate *HR_{target}* constitutes the target heart rate range; and
a fourth target heart rate range calculation unit, configured to: if the heart rate information of the user is that the resting heart rate is tested and the medication information is that the medication for slowing down the heart rate is used, calculate the target heart rate *HR_{target}* of the user based on the maximum heart rate *HRₘₐₓ*, the expected intensity *ES* of the recommended exercise, and the resting heart rate *RHR* of the user by using the following formula: *HR_{target}* = (*HRₘₐₓ* - *RHR)* × (*ESₘᵢₙ* - 10%, *ESₘₐₓ* - 5%) + RHR; where *ESₘᵢₙ* represents a lower limit of the expected intensity *ES* of the recommended exercise, and *ESₘₐₓ* represents an upper limit of the expected intensity *ES* of the recommended exercise; and the value range of the target heart rate *HR_{target}* constitutes the target heart rate range.

In practical use, the user first downloads a user-side application, registers and logs in by using a mobile phone number, and fills in a health questionnaire. The health data acquisition module acquires health questionnaire information. The medication data acquisition module acquires the medication information of the user. The user wears the heart rate detection device. The exercise test data acquisition module acquires the heart rate information. All the data acquisition modules input the data to the core processor through the data input module. The core processor calculates the target heart rate range of the user by using different formulas based on whether the user tests the resting heart rate in an exercise test, whether the user uses the medication for slowing down the heart rate, and the age and the health condition of user. The target heart rate range is recommended to the doctor end. A doctor checks the target heart rate range recommended by the system through the doctor-side target heart rate display module, and clicks a sending button. The target heart rate range is sent to the user end through the data sending module and displayed through the user-side target heart rate display module. The data information is stored in data storage modules of the doctor end, the user end, and the server. The apparatus for differentially calculating target heart rates of different users in the present disclosure can accurately and personalizedly recommend target heart rate ranges based on different user conditions to make exercise more scientific and targeted.

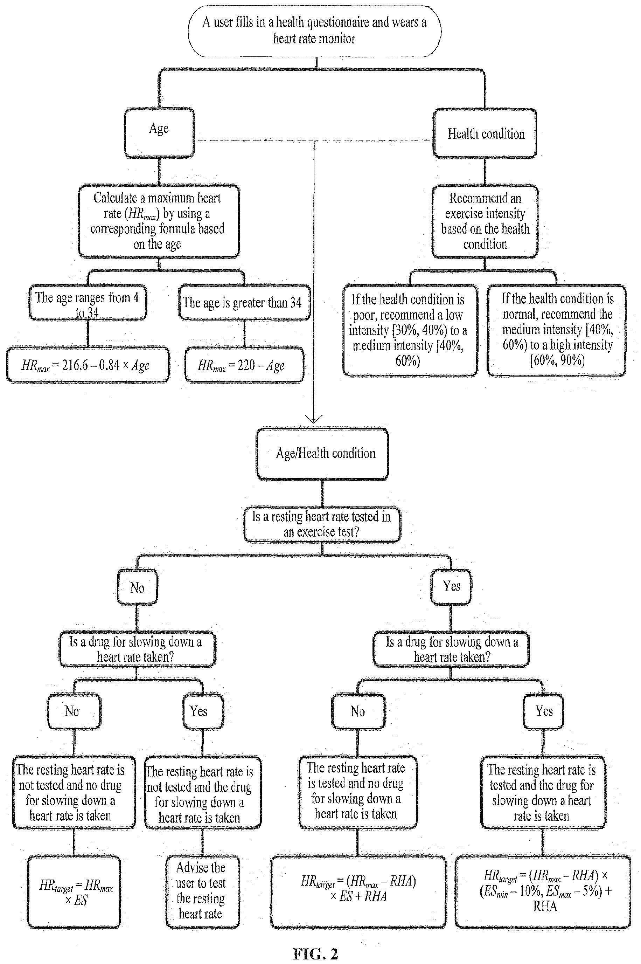

FIG. 2 is a schematic diagram of an application process of an apparatus for differentially calculating target heart rates of different users according to an embodiment of the present disclosure. Referring to FIG. 2, the following uses a specific embodiment to describe the application process of the apparatus for differentially calculating target heart rates of different users in the present disclosure. The application process specifically includes:
(1) A user downloads a user-side application, registers and logs in by using a mobile phone number, and fills in a health questionnaire. Questionnaire information includes but is not limited to an age, a gender, a height, a weight, a disease diagnosis result, smoking, drinking, an exercise habit, and whether the user uses a medication for slowing down heart rate.
(2) A health data acquisition module acquires the health questionnaire information. A data input module inputs the acquired health information to a core processor.
(3) A medication data acquisition module acquires medication information of the user. The data input module inputs the acquired medication information to the core processor.
(4) The user wears a heart rate detection device, including but not limited to a device with a heart rate detection function, such as a heart rate monitor, a smart watch, or a smart band, and connected to an exercise test data acquisition module. The data input module inputs acquired heart rate information to the core processor.
(5) The core processor performs data analysis and processing through the following logic:
   (5.1) Age (*Age*): Calculate a maximum heart rate (*HRₘₐₓ*) of the user by using a corresponding formula based on the age of the user. If the age of the user ranges from 4 to 34, use the following formula: *HRₘₐₓ* = 216.6 - 0.84 × *Age.* For example, if the age of the user is 18, *HRₘₐₓ* = 216.6 - 0.84 × 18 ≈ 201.5 beats per minute. If the age of the user is greater than 34, use the following formula: *HRₘₐₓ =* 220 - *Age.* For example, if the age of the user is 50, *HRₘₐₓ =* 220 - 50 = 170 beats per minute.
   (5.2) Health condition: Recommend an exercise intensity (namely, an expected intensity *ES* of recommended exercise) based on the health condition of the user. If the health condition of the user is poor, recommend a low intensity (greater than and equal to 30% and less than 40%) to a medium intensity (greater than and equal to 40% and less than 60%). For example, the recommended exercise intensity is 30% to 60% for users (or patients) with pneumoconiosis, 40% to 60% for users (or patients) with coronary heart disease, and 40% to 60% for users (or patients) with breast cancer. If the health condition of the user is normal, recommend the medium intensity (greater than and equal to 40% and less than 60%) to a high intensity (greater than and equal to 60% and less than 90%). For example, the recommended exercise intensity is 40% to 80% for users with sub-health.
   (5.3) Use different formulas to calculate a target heart rate range of the user based on whether the user tests a resting heart rate in an exercise test, whether the user uses a medication for slowing down heart rate, and the age and the health conditions of the user, and store the target heart rate range in a data storage module of a server. The following four cases are involved:
      1) If the user does not test the resting heart rate and does not use the medication for slowing the heart rate, use the following formula: Target heart rate (*HR_{target}*) = Maximum heart rate × Expected intensity (the expected intensity is a percentage). For example, if the age of the user is 18, and the user has sub-health, does not test the resting heart rate, and does not use the medication for slowing down the heart rate, *HR_{target}* = (216.6 - 0.84 × 18) × (40% to 80%). In this case, the target heart rate range is (80.6,161.2). If the age of the user is 50, and the user has breast cancer, does not test the resting heart rate, and does not use the medication for slowing down the heart rate, *HR_{target} =* (220 - 50) × (40% to 60%). In this case, the target heart rate range is (68,102).
      2) If the user does not test the resting heart rate and uses the medication for slowing down the heart rate, advise the user to test the resting heart rate for safety.
      3) If the user tests the resting heart rate and does not use the medication for slowing the heart rate, use the following formula: Target heart rate (*HR_{target}*) = (Maximum heart rate - Resting heart rate) × Expected intensity + Resting heart rate. For example, if the age of the user is 18, the resting heart rate of the user is 70 beats per minute, and the user has sub-health and does not use the medication for slowing down the heart rate, *HR_{target} =* [(216.6 - 0.84 × 18) - 70] × (40% to 80%) + 70. In this case, the target heart rate range is (122.6,175.2). If the age of the user is 50, the resting heart rate of the user is 80 beats per minute, and the user has breast cancer and does not use the medication for slowing down the heart rate, *HR_{target} =* [(220 - 50) - 80] × (40% to 60%) + 80. In this case, the target heart rate range is (116,134).
      4) If the user tests the resting heart rate and uses the medication for slowing the heart rate, use the following formula: Target heart rate (*HR_{target}*) = (Maximum heart rate - Resting heart rate) × Expected intensity (Lower limit - 10%, Upper limit - 5%) + Resting heart rate. For example, if the age of the user is 50, the resting heart rate of the user is 80 beats per minute, and the user has coronary heart disease and uses the medication for slowing down the heart rate, *HR_{target} =* [(220 - 50) - 80] × [(40% - 10%) to (60% - 5%)]+80. In this case, the target heart rate range is (107,129.5).
      (6) The core processor recommends a data analysis result (namely, the target heart rate range) to a doctor end. A doctor checks the target heart rate range recommended by the system through a doctor-side target heart rate display module and clicks a sending button. The target heart rate range is sent to the user end through the data sending module and displayed through a user-side display module. The data information is stored in data storage modules of the doctor end and the user end.

In the present disclosure, the three acquisition modules, namely, the health data acquisition module, the medication data acquisition module, and the exercise test data acquisition module are designed to acquire the health information, the medication information, and the heart rate information of the user. In the present disclosure, the data input module inputs all the acquired information to the core processor for data processing. The core processor uses different formulas to calculate target heart rates of different users based on different information of the users (including the age, the health condition, whether the user tests the resting heart rate in an exercise test, whether the user uses the medication for slowing down the heart rate, and the like). The analysis result of the core processor is sent to the doctor end. After the doctor checks the target heart rate range through the target heart rate display module, the target heart rate range is sent to the user end through the data sending module and displayed through the user-side target heart rate display module. All data analysis results are stored in the data storage modules of the doctor end and the user end.

In the present disclosure, the foregoing design is used to accurately and personalizedly recommend target heart rate ranges based on different user conditions. Appropriate exercise prescriptions can be formulated based on the target heart rate ranges such that exercise can be more scientific and targeted.

Each embodiment of the present specification is described in a progressive manner, each embodiment focuses on the difference from other embodiments, and the same and similar parts between the embodiments may refer to each other. Since the system disclosed in an embodiment corresponds to the method disclosed in another embodiment, the description is relatively simple, and reference can be made to the method description.

Specific examples are used herein to explain the principles and embodiments of the present disclosure. The foregoing description of the embodiments is merely intended to help understand the method of the present disclosure and its core ideas; besides, various modifications may be made by a person of ordinary skill in the art to specific embodiments and the scope of application in accordance with the ideas of the present disclosure. In conclusion, the content of the present description shall not be construed as limitations to the present disclosure.

## Claims

1. An apparatus for differentially calculating target heart rates of different users, comprising: a user client, a heart rate detection device, a server, and a doctor client; wherein the user client comprises a health data acquisition module, a medication data acquisition module, and a user-side target heart rate display module; the heart rate detection device is worn on a user and comprises an exercise test data acquisition module; the server comprises a data input module and a core processor; and the doctor client comprises a data sending module and a doctor-side target heart rate display module;
the health data acquisition module is configured to acquire health information of the user; the health information comprises an age and a health condition of the user; and the health condition comprises a disease diagnosis result of the user;
the medication data acquisition module is configured to acquire medication information of the user; and the medication information comprises whether the user uses medications for slowing down a heart rate;
the exercise test data acquisition module is configured to acquire heart rate information of the user; and the heart rate information comprises whether the user tests a resting heart rate and the resting heart rate of the user if the user tests the resting heart rate;
the data input module is configured to input the health information, the medication information, and the heart rate information of the user to the core processor;
the core processor is configured to calculate a target heart rate range of the user based on the health information, the medication information, and the heart rate information of the user and send the target heart rate range to the doctor client;
the doctor-side target heart rate display module is configured to display the target heart rate range on the doctor client;
the data sending module is configured to send the target heart rate range from the doctor client to the user client; and
the user-side target heart rate display module is configured to display the target heart rate range on the user client.

2. The apparatus for differentially calculating target heart rates of different users according to claim 1, wherein the core processor specifically comprises:
a maximum heart rate calculation submodule, configured to calculate a maximum heart rate of the user based on the age of the user;
an expected intensity determining submodule, configured to determine an expected intensity of recommended exercise of the user based on the health condition of the user; and
a target heart rate range calculation submodule, configured to calculate the target heart rate range of the user based on the maximum heart rate, the expected intensity of the recommended exercise, the medication information, and the heart rate information of the user and send the target heart rate range to the doctor client.

3. The apparatus for differentially calculating target heart rates of different users according to claim 2, wherein the maximum heart rate calculation submodule specifically comprises:
a first maximum heart rate calculation unit, configured to: if the age (*Age*) of the user ranges from 4 to 34, calculate the maximum heart rate (*HRₘₐₓ)* of the user by using the following formula: *HRₘₐₓ =* 216.6 - 0.84 × *Age*; and
a second maximum heart rate calculation unit, configured to: if the age (*Age*) of the user is greater than 34, calculate the maximum heart rate (*HRₘₐₓ*) of the user by using the following formula: *HRₘₐₓ =* 220 - *Age.*

4. The apparatus for differentially calculating target heart rates of different users according to claim 3, wherein the expected intensity determining submodule specifically comprises:
a first expected intensity determining unit, configured to: if the health condition of the user is poor, determine the expected intensity *(ES)* of the recommended exercise of the user as a low intensity to a medium intensity; wherein the low intensity is greater than or equal to 30% and less than 40%; and the medium intensity is greater than or equal to 40% and less than 60%; and
a second expected intensity determining unit, configured to: if the health condition of the user is normal, determine the expected intensity *(ES)* of the recommended exercise of the user as the medium intensity to a high intensity; wherein the high intensity is greater than or equal to 60% and less than 90%.

5. The apparatus for differentially calculating target heart rates of different users according to claim 4, wherein the target heart rate range calculation submodule specifically comprises:
a first target heart rate range calculation unit, configured to: if the heart rate information of the user is that the resting heart rate is not tested and the medication information is that no medication for slowing down the heart rate is used, calculate a target heart rate (*HR_{target}*) of the user based on the maximum heart rate (*HRₘₐₓ)* and the expected intensity *(ES)* of the recommended exercise of the user by using the following formula: *HR_{target} = HRₘₐₓ* × *ES*; wherein a value range of the target heart rate (*HR_{target})* constitutes the target heart rate range.

6. The apparatus for differentially calculating target heart rates of different users according to claim 5, wherein the target heart rate range calculation submodule further comprises:
a second target heart rate range calculation unit, configured to: if the heart rate information of the user is that the resting heart rate is not tested and the medication information is that the medication for slowing down the heart rate is used, remind, through the user-side target heart rate display module, the user to test the resting heart rate.

7. The apparatus for differentially calculating target heart rates of different users according to claim 6, wherein the target heart rate range calculation submodule further comprises:
a third target heart rate range calculation unit, configured to: if the heart rate information of the user is that the resting heart rate is tested and the medication information is that no medication for slowing down the heart rate is used, calculate the target heart rate (*HR_{target}*) of the user based on the maximum heart rate (*HRₘₐₓ*), the expected intensity *(ES)* of the recommended exercise, and the resting heart rate (*RHR)* of the user by using the following formula: *HR_{target}* = (*HRₘₐₓ* - *RHR)* × *ES* + *RHR*; wherein the value range of the target heart rate (*HR_{target}*) constitutes the target heart rate range.

8. The apparatus for differentially calculating target heart rates of different users according to claim 7, wherein the target heart rate range calculation submodule further comprises:
a fourth target heart rate range calculation unit, configured to: if the heart rate information of the user is that the resting heart rate is tested and the medication information is that the medication for slowing down the heart rate is used, calculate the target heart rate (*HR_{target})* of the user based on the maximum heart rate (*HRₘₐₓ)*, the expected intensity *(ES)* of the recommended exercise, and the resting heart rate *(RHR)* of the user by using the following formula: *HR_{target}* = (*HRₘₐₓ* - *RHR)* × (*ESₘᵢₙ* - 10%, *ESₘₐₓ* - 5%) + RHR; wherein *ESₘᵢₙ* represents a lower limit of the expected intensity *(ES)* of the recommended exercise, and *ESₘₐₓ* represents an upper limit of the expected intensity *ES* of the recommended exercise; and the value range of the target heart rate (*HR_{target}*) constitutes the target heart rate range.
